# DEMANDE DE BREVET EUROPEEN

(11) **EP 3 143 955 A1**
(43) Date de publication de la demande: **22.03.2017**
(21) Numéro de dépôt: 16185971.5
(22) Date de dépôt: 02.10.2009
(51) Int. Cl.: A61B 17/80

(54) **IMPLANT ORTHOPEDIQUE SOUS FORME D'UNE PLAQUE DESTINEE A ETRE FIXEE ENTRE DEUX PARTIES D'OS**

(30) Priorité: 02.10.2008 FR 0856693
(62) Demande divisionnaire de: 09756158.3
(71) Demandeur: Stryker European Holdings I, LLC, Kalamazoo, MI 49002 (US)
(72) Inventeur: PRANDI, Bernard, 35700 RENNES (FR); DELMI, Marino, CH-1231 CONCHES (CH); COETZEE, J. Chris, EAGAN, MN Minnesota 55122 (US); WELL, Lowell, JR, LAKE FOREST, IL Illinois 60045 (US); WAPNER, Keith., Philadelphia, PA Pennsylvania 19106 (US); CHIODO, Christopher P., JAMAICA PLAIN, MA Massachusetts 02130 (US); COUGHLIN, Michael, BOISE, ID Idaho 83706 (US); TOURNE, Yves, 38610 GIERES (FR); WALKER, Christophe Richard, HESWALL LOIRRAL, CH60 4SE (GB); BOHAY, Donald R., ADA, MI Michigan 49301 (US); ANDERSON, John G., LOWELL, MI Michigan 49331 (US)
(74) Mandataire: Regimbeau

(57) **Abrégé**

L'invention porte sur une plaque pour arthrodèse ou ostéosynthèse ayant une longueur adaptée pour s'étendre entre une première (O1) et une deuxième (02) partie d'os, la plaque ayant un premier logement (1c) pour l'introduction d'une première broche de fixation (4) du côté de la première partie d'os (O1) et un deuxième logement (1d) pour l'introduction d'une deuxième broche de fixation (5) du côté de la deuxième partie d'os (02), caractérisé en ce que le premier logement (1c) est un trou circulaire ayant un diamètre correspondant sensiblement à un diamètre de la première broche de fixation (4), et le deuxième logement (1d) est une lumière oblongue ayant une largeur correspondant sensiblement à un diamètre de la deuxième broche de fixation (5).

## Description

L'invention se rattache au secteur technique des implants orthopédiques.

Plus particulièrement, l'invention concerne une plaque pour arthrodèse ou ostéosynthèse destinée à être fixée entre deux parties d'os.

D'une manière parfaitement connue pour un homme du métier, ce type de plaque comprend, généralement, des trous pour l'engagement de vis permettant de réaliser une arthrodèse entre deux os ou une ostéosynthèse entre deux fragments osseux. C'est le cas, par exemple, pour les os de la main ou du pied, sans pour cela exclure d'autres applications, notamment dans le domaine du rachis. En fonction du cas pathologique à traiter, ces plaques peuvent être de forme générale rectiligne ou présenter d'autres formes géométriques.

A partir de cet état de la technique, l'un des problèmes que se propose de résoudre l'invention est d'améliorer, d'une manière sure et efficace, la compression entre les parties d'os assujetties à la plaque et selon une direction précise.

Pour résoudre un tel problème, il a été conçu et mis au point un implant orthopédique sous forme d'une plaque destinée à être fixée entre deux parties d'os au moyen de vis engagées dans des trous formés dans l'épaisseur de ladite plaque.

Selon l'invention, les agencements de la plaque aptes à assurer, sous un effet de vissage, un effort de compression réglable pour rapprocher les deux parties d'os, sont constitués par au moins un logement qui présente un profil en section délimitant plusieurs zones distinctes aptes à coopérer avec une vis, afin de générer un déplacement progressif en translation de la vis au fur et à mesure d'un vissage correspondant à un déplacement de l'une des parties d'os au moins, engendrant l'effort de compression.

Pour résoudre le problème posé d'obtenir une compression maîtrisée et réglable, l'une des zones est constituée par un trou de diamètre D1 destiné à recevoir la tête de la vis, tandis qu'une autre zone est constituée par un trou de diamètre D2 inférieur au diamètre D1, et présentant, sur une hauteur déterminée, une pente comprise entre 40 et 60°, lesdites zones étant reliées par une zone intermédiaire présentant une pente en direction de la zone constituée par le trou de diamètre D1.

Avantageusement, la pente de la zone intermédiaire est comprise entre 15 et 30°.

Un autre problème que se propose de résoudre l'invention est d'assurer une fixation temporaire de la plaque pour faciliter la fixation de cette dernière par l'opérateur au moyen de broches et de permettre, après mise en place des broches dans l'une des parties d'os, de coulisser sous la plaque au moment du vissage tout en assurant une compression selon une direction précise.

Pour résoudre un tel problème, la plaque présente, d'une manière connue, un logement pour l'introduction d'une broche du côté de l'une des parties d'os et un autre logement pour l'introduction d'une broche du côté de l'autre partie d'os.

Selon l'invention, l'un des logements est constitué par un trou circulaire dont le diamètre correspond sensiblement à celui de la broche, tandis que l'autre logement est constitué par une lumière oblongue.

Pour résoudre le problème posé de respecter l'anatomie notamment dans le cas d'une plaque d'arthrodèse MTP, l'axe de la lumière oblongue est orienté angulairement par rapport à l'axe longitudinal de la plaque selon un angle compris entre 1 et 10° environ.

D'une manière générale, la plaque présente des trous lisses et/ou taraudés destinés à recevoir des vis de fixation avec les parties d'os.

A partir de cette conception de base de la plaque et du cas pathologique à traiter :
▪ soit les différents trous sont alignés;
▪ soit certains des trous sont disposés selon le sommet d'un triangle ou d'un quadrilatère.

Dans une forme de réalisation, la plaque est centrée longitudinalement pour s'adapter à la courbure des parties d'os.

L'invention est exposée ci-après plus en détail à l'aide des figures des dessins annexés dans lesquels :
- la figure 1 est une vue en perspective d'une forme de réalisation d'une plaque selon l'invention;
- la figure 2 est une vue partielle en perspective à grande échelle montrant le logement de compression;
- la figure 3 est une vue en coupe longitudinale correspondant à la figure 2 ;
- les figures 4, 5 et 6 sont des vues partielles en perspective, en coupe, à caractère schématique montrant le principe de la compression obtenue au fur et à mesure de l'engagement de la vis dans le logement de compression;
- la figure 7 est une vue en perspective d'une autre forme de réalisation de la plaque, notamment pour arthrodèse MTP ;
- les figures 8, 9 et 10 sont des vues en perspective d'autres formes géométriques de la plaque données à titre indicatif;
- la figure 11 montre une forme de réalisation de la plaque profilée longitudinalement pour s'adapter à la courbure de l'os;
- les figures 12 à 18 sont des vues en perspective montrant le principe de mise en place et de fixation de la plaque par rapport à deux parties d'os en vue d'assurer la compression de ces dernières, lesdites parties d'os étant représentées schématiquement par des blocs de forme générale parallélépipédique ;
- la figure 19 est une vue partielle de la plaque montrant, de manière schématique, deux positions extrêmes du perçage initial pour obtenir une compression maximale (position 1) ou une compression nulle (position 0).

La plaque d'ostéosynthèse et/ou d'arthrodèse est désignée dans son ensemble par (1). D'une manière connue, cette plaque (1) présente des trous lisses et/ou taraudés (1a) pour l'engagement de vis de fixation (2) vissées dans les parties d'os (01) et (02), comme il sera indiqué dans la suite de la description.

Selon une caractéristique à la base de l'invention, la plaque (1) présente au moins un logement ou plot de compression (1b).

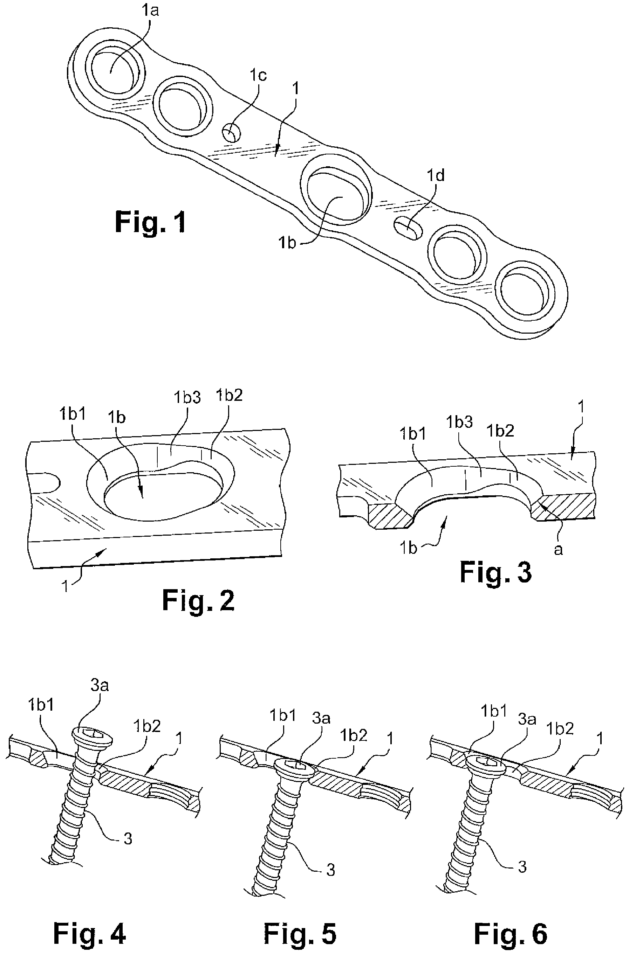

Comme le montrent notamment les figures 2 et 3, ce logement (1b) présente plusieurs zones distinctes (1b1), (1b2) et (1b3), afin de permettre de régler la compression désirée, notamment en fonction de la qualité osseuse, en fonction de la position de départ d'une vis (3) dans ledit logement.

Plus particulièrement, le logement (1b) est constitué par trois zones distinctes qui présentent les caractéristiques suivantes :
- La zone (1b1) est constituée par un trou de diamètre D1 destiné à recevoir la tête (3a) de la vis (3).
- La zone (1b2) est constituée par un trou de diamètre D2 inférieur au diamètre D1 présentant, sur une hauteur déterminée, comprise entre 1 et 2 mm environ, une pente de l'ordre de 40 à 60°, par rapport au plan de base de la plaque.
- Les zones (1b1) et (1b2) sont reliées par une zone intermédiaire de transition (1b3) présentant une pente descendante, c'est-à-dire dirigée en direction de la zone (1b1). Cette pente est comprise entre 15 et 30° environ.

Compte tenu de ces caractéristiques, la mise en compression des deux parties d'os (01) et (02) s'effectue comme suit, en référence notamment aux figures 4, 5, 6, 15 et 16.
- Un trou est préalablement réalisé dans l'une des parties d'os considérée selon la zone (1b2) ou (1b1) en fonction de la compression que l'on veut obtenir.
- La vis (3) est insérée dans le trou au contact de la tête (3a) sur la plaque (1) (figures 4 et 5).
- Le trou de la zone (1b2) étant de diamètre inférieur à celui de la tête de vis (3), cette dernière vient immédiatement se décaler au-delà de l'axe de la zone (1b1) entraînant une première mise en compression rapide.
- En poursuivant l'action de vissage, la tête de vis (3a) est vissée le long de la pente de la zone intermédiaire (1b3) entraînant une seconde mise en compression jusqu'à trouver une position de stabilité au niveau du trou (1b1) dont le diamètre est adapté à celui de la tête de vis (3a). Bien évidemment, il est possible de s'arrêter en toute position intermédiaire, afin d'obtenir un réglage précis de la compression.

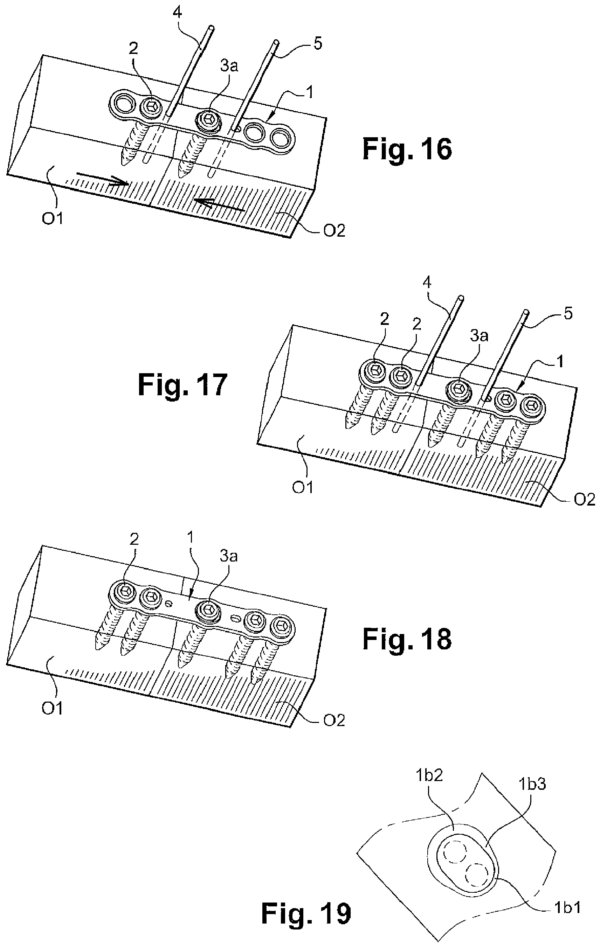

A noter également que le niveau de compression généré est de l'ordre de 0 à 3 mm et peut être maîtrisé en fonction de la localisation du passage initial. Ainsi, il est possible d'obtenir une compression maximale pour un logement situé en position 1 et une absence de compression pour un logement situé dans une position 0 (figure 19).

Selon une autre caractéristique, la plaque (1) présente au moins un logement (1c) pour l'introduction d'une broche (4) en vue d'assurer une fixation temporaire de ladite plaque (1). Avantageusement, la plaque (1) présente un logement (1c) pour l'introduction d'une broche (3) du côté de l'une des parties de l'os (01) et autre logement (1d) pour l'introduction d'une autre broche (5) du côté de l'autre partie d'os (02).

Compte tenu de l'effet de compression recherchée, telle qu'indiquée précédemment, le logement (1c) est constitué par un trou circulaire dont le diamètre correspond sensiblement à celui de la broche (4), tandis que l'autre logement (1d) est constitué par une lumière oblongue, situé du côté du logement de compression (1b).

Ces dispositions permettent donc à l'os de coulisser sous la plaque (1) au moment du vissage, tout en assurant une compression selon une direction précise, généralement suivant l'axe de la plaque. Les broches (4) et (5) sont de tout type connu et approprié, et parfaitement connu pour un homme du métier.

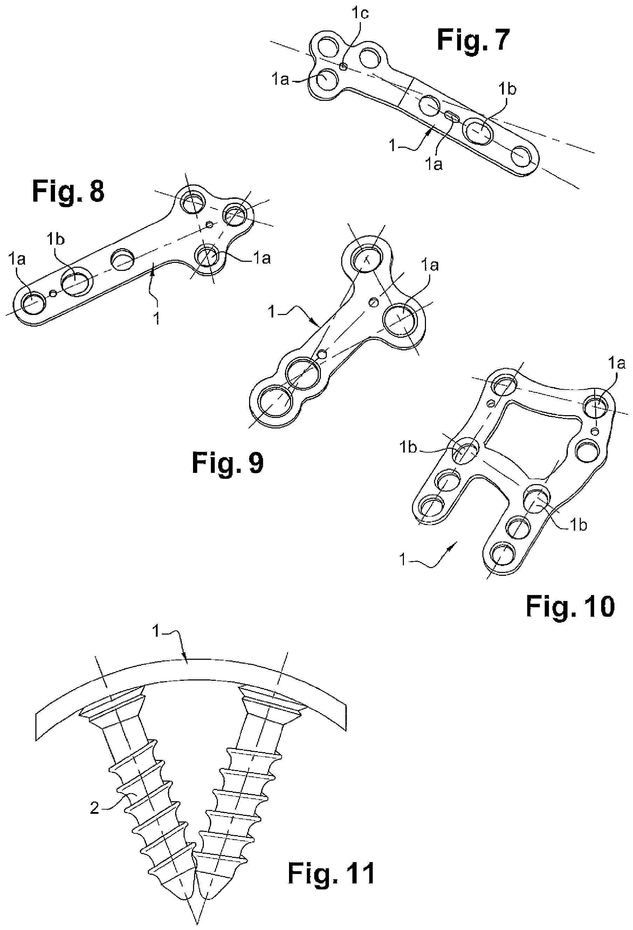

La lumière oblongue (1d) de fixation temporaire est particulièrement bien adaptée dans le cas d'une plaque d'arthrodèse MTP afin de respecter l'anatomie selon laquelle l'angle entre la phalange P1 et le métatarse 1 est de l'ordre de 10°. En effet, une plaque connue selon l'état de la technique permet difficilement d'assurer la compression tout en gardant l'angulation précitée. Selon l'invention, il suffit que la lumière (1d) soit orientée angulairement par rapport à l'axe médian de la plaque (1) selon un angle d'environ plus ou moins 5 degrés (figure 7).

Comme le montrent les figures des dessins, la plaque (1) peut présenter différentes formes géométriques, de sorte que les trous (1a) notamment peuvent être alignés (figure 1) ou être disposés, en totalité ou en partie, selon les sommets d'un triangle ou d'un quadrilatère (figures 8, 9 et 10). Plus particulièrement, à la figure 8, la plaque est conformée pour être utilisée dans le cas d'une arthrodèse du Lapidus, à la figure 9, pour une ostéotomie basale du premier métatarse et pour une arthrodèse du Lisfranc 2/3 (figure 10). Ces dispositions, en triangle ou en quadrilatère des vis, améliorent la stabilité du montage.

A noter également que la plaque (1), quelle que soit sa forme géométrique, peut être cintrée longitudinalement, pour s'adapter à la courbure de l'os (figure 6) permettant, en conséquence, aux vis (2) de former un angle entre elles (figure 11).

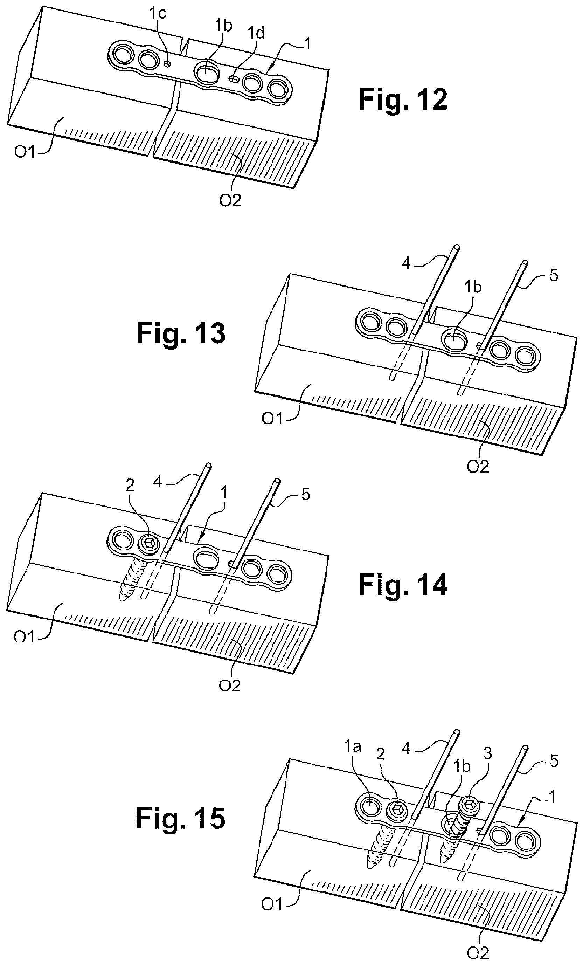

On renvoie aux figures 12 à 18 qui expliquent la mise en place de la plaque selon l'invention :
▪ Après réalisation des ostéotomies, la plaque (1) est positionnée entre les deux parties d'os (01) et (02) (figure 12).
▪ Le chirurgien stabilise la plaque (1) au moyen des deux broches temporaires (4) et (5) dont l'une est engagée dans le trou (1c), tandis que l'autre est engagé dans la lumière (1d) (figure 13).
▪ Au moins une vis (2) est engagée dans le trou (1a) pour être vissée dans la partie d'os (01) où n'est pas positionné le plot de compression (1b) (figure 14).
▪ Le chirurgien visse ensuite, dans le plot de compression (1b), la vis (3), en choisissant le taux de compression, comme indiqué précédemment (figure 15).
▪ Lorsque la compression est effectuée (figure 16), le chirurgien peut visser une ou plusieurs vis complémentaires (2) (figure 17), puis retirer les broches de guidage (4) et (5) (figure 18).

Les avantages ressortent bien de la description ci-dessus ainsi que des exemples numérotés ci-dessous.

L'exemple 1 concerne un implant orthopédique sous forme d'une plaque (1) destinée à être fixée entre deux parties d'os au moyen de vis (3) engagées dans des trous (1a) formés dans l'épaisseur de ladite plaque qui présente des agencements aptes à assurer, sous un effet de vissage, un effort de compression réglable apte à rapprocher les deux parties d'os, dans lequel les agencements sont constitués par au moins un logement (1b) qui présente un profil en section délimitant plusieurs zones distinctes (1b1), (1b2) et (1b3) aptes à coopérer avec une vis (3), afin de générer un déplacement progressif en translation de la vis (3) au fur et à mesure d'un vissage correspondant à un déplacement de l'une des parties d'os au moins, engendrant l'effort de compression, ladite plaque (1) présentant un logement (1c) pour l'introduction d'une broche (4) du côté de l'une des parties d'os et un autre logement (1d) pour l'introduction d'une broche (5) du côté de l'autre partie d'os, l'un des logements (1c) est constitué par un trou circulaire dont le diamètre correspond sensiblement à celui de la broche (4), tandis que l'autre logement (1d) est constitué par une lumière oblongue.

L'exemple 2 concerne un implant selon l'exemple 1, dans lequel une des zones est constituée par un trou (1b1) de diamètre D1 destiné à recevoir la tête (3a) de la vis (3), tandis qu'une autre zone est constituée par un trou (1b2) de diamètre D2 inférieur au diamètre D1, et présentant, sur une hauteur déterminée, une pente comprise entre 40 et 60° par rapport au plan de base de la plaque, lesdites zones (1b1) et (1b2) étant reliées par une zone intermédiaire (1b3) présentant une pente en direction de la zone constituée par le trou de diamètre D1.

L'exemple 3 concerne un implant selon l'exemple 1, dans lequel la pente de la zone intermédiaire (1b3) est comprise entre 15 et 30°.

L'exemple 4 concerne un implant selon l'exemple 1, dans lequel l'axe de la lumière oblongue est orienté angulairement par rapport à l'axe longitudinal de la plaque selon un angle compris entre 1 et 15° environ.

L'exemple 5 concerne un implant selon l'un quelconque des exemples 1 à 4, dans lequel la plaque (1) présente des trous lisses et/ou taraudés destinés à recevoir des vis de fixation (2) et (3) avec les parties d'os.

L'exemple 6 concerne un implant selon l'exemple 5, dans lequel les différents trous sont alignés.

L'exemple 7 concerne un implant selon l'exemple 5, dans lequel certains des trous sont disposés selon le sommet d'un triangle ou d'un quadrilatère.

L'exemple 8 concerne un implant selon l'exemple 1, dans lequel la plaque (1) est cintrée longitudinalement pour s'adapter à la courbure des parties d'os.

## Revendications

1. Plaque pour arthrodèse ou ostéosynthèse ayant une longueur adaptée pour s'étendre entre une première (01) et une deuxième (02) partie d'os, la plaque ayant un premier logement (1c) pour l'introduction d'une première broche de fixation (4) du côté de la première partie d'os (O1) et un deuxième logement (1d) pour l'introduction d'une deuxième broche de fixation (5) du côté de la deuxième partie d'os (02), **caractérisé en ce que** le premier logement (1c) est un trou circulaire ayant un diamètre correspondant sensiblement à un diamètre de la première broche de fixation (4), et le deuxième logement (1d) est une lumière oblongue ayant une largeur correspondant sensiblement à un diamètre de la deuxième broche de fixation (5).

2. Plaque selon la revendication 1, dans laquelle la lumière oblongue est agencée de sorte à avoir un axe longitudinal orienté angulairement par rapport à un axe longitudinal de la plaque.

3. Plaque selon la revendication 2, dans laquelle l'axe longitudinal de la lumière oblongue est orienté selon un angle compris entre 1° et 10° par rapport à l'axe longitudinal de la plaque.

4. Plaque selon la revendication 2, dans laquelle l'axe longitudinal de la lumière oblongue est orienté selon un angle de 5° par rapport à l'axe longitudinal de la plaque.

5. Plaque selon l'une quelconque des revendications 1 à 4, comprenant en outre des moyens de mise en compression prévu pour rapprocher les première (01) et deuxième (02) parties d'os sous un effet de vissage.

6. Plaque selon la revendication 5, dans laquelle les moyens de compression comprennent au moins un logement de compression (1b) présentant un profil en section délimitant plusieurs zones distinctes (1b1, 1b2, 1b3) aptes à coopérer avec une vis (3) pour régler une compression désirée en fonction de la position de la vis (3) dans ledit logement de compression (1b).

7. Plaque selon la revendication 6, dans laquelle le logement de compression (1b) et le deuxième logement (1d) ont chacun un axe central s'étendant dans la deuxième partie d'os (02) sans s'étendre dans la première partie d'os (01).

8. Plaque selon l'une quelconque des revendications 1 à 7, dans laquelle la plaque comprend une pluralité de logements agencés selon les sommets d'un triangle ou d'un quadrilatère, une partie de la pluralité des logements étant alignée avec la première partie d'os (01) et une partie de la pluralité des logements étant alignée avec la deuxième partie d'os (02).

9. Plaque selon l'une quelconque des revendications 1 à 8, dans laquelle la plaque est cintrée longitudinalement pour s'adapter à la courbure des première (01) et deuxième (02) parties d'os.

10. Plaque selon la revendication 9, dans laquelle la courbure de la plaque permet aux première (4) et deuxième (5) broches de fixation de former un angle entre elles lorsqu'elles sont insérées dans les premier (1c) et deuxième (1d) logements respectivement.
